# EUROPEAN PATENT SPECIFICATION

(11) **EP 1 493 690 B1**
(45) Date of publication and mention of the grant of the patent: **18.10.2006**
(21) Application number: 04253464.4
(22) Date of filing: 10.06.2004
(51) Int. Cl.: B65G 15/20, B65G 29/02

(54) **Belt drive assembly for container inspection machine**
Riemenantrieb für einen Behälterinspektionssystem
Ensemble d'entraînement par courroie pour dispositif d'inspection de récipients

(30) Priority: 30.06.2003 US 610233
(43) Date of publication of application: 05.01.2005
(73) Proprietor: Emhart Glass S.A., 6330 Cham (CH)
(72) Inventor: Raupp, Henry F., Freeville, New York 13068 (US)
(74) Representative: Warren, Anthony Robert

(56) References cited:
- US-A- 3 901 381
- US-A- 5 422 476
- US-A- 5 492 216
- US-A- 5 624 021
- US-A- 5 823 317
- US-A- 6 109 426
- US-B1- 6 250 851
- US-B1- 6 390 282

## Description

The present invention relates to machines which inspect containers such as bottles for defects, and more particularly to such machines wherein a bottle is conveyed through one or more inspection stations via a belt conveyor having opposed pairs of belts.

A state of the art glass bottle inspection machine wherein the bottles are transported through a number of inspection stations by a belt drive mechanism having opposed pairs of horizontal belts is disclosed in US-A-5,422,476.

It is an object of the present invention to provide an improved belt drive for such machines.

According to the present invention, there is provided a belt drive conveyor system for a machine for inspecting containers as defined in claim 1.

The invention also consists in a machine for inspecting containers incorporating a conveyor system as above defined.

The present invention will now be described with reference to the accompanying drawings which illustrate a presently preferred embodiment incorporating the principles of the invention, and in which:
Figure 1 is a schematic oblique view of an inspection machine for inspecting bottles made in accordance with the teachings of the present invention;
Figure 2 is an oblique view of the belt drive assembly of the machine illustrated in Figure 1, in its large ware configuration;
Figure 3 is an oblique view of a guide wheel assembly for one of the opposed belt drives;
Figure 4 is an oblique view of the belt drive assembly housing including the open/close step motors;
Figure 5 is a bottom view of a belt drive unit; and
Figure 6 is an oblique view of the belt drive assembly illustrated in Figure 2, in its mini ware configuration.

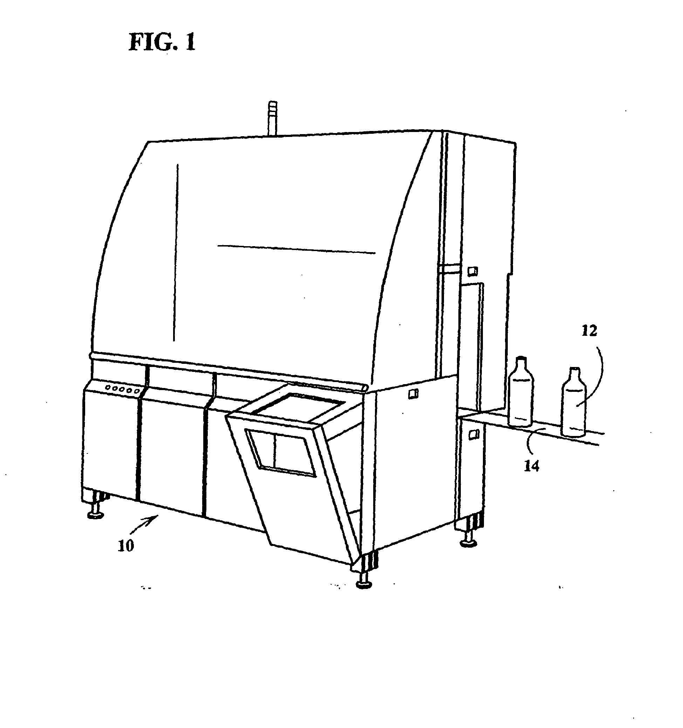

Figure 1 is a schematic showing of an inspection machine 10 which inspects a row of bottles 12 conveyed to the machine by an infeed conveyor 14. Any of a variety of inspections can be carried out by the machine.

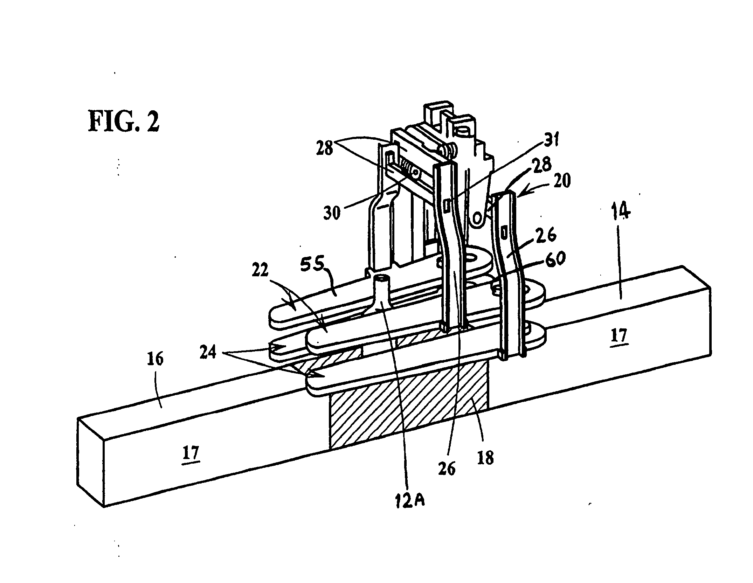

Figure 2 illustrates the belt conveyor mechanism 20 for this machine which receives, i.e. picks up, bottles from the infeed conveyor 14, conveys them through one or more inspection locations, and releases them to a discharge conveyor 16. The infeed and discharge conveyors have front guards 17 which, together with a front guard 18 for the inspection equipment (light sources, cameras, etc.), define the front face of the infeed/discharge conveyor system.

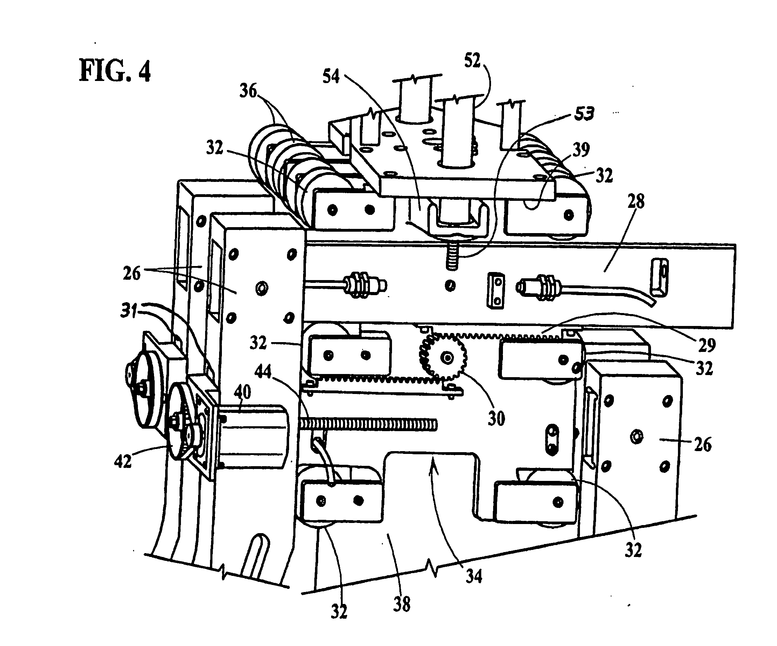

As can be seen, the belt conveyor mechanism has an upper opposed pair of belt drives 22 and a lower opposed pair of belt drives 24. Each of the belt drives 22, 24 is cantilevered from a generally vertical or upwardly extending strut 26 which is integral with a horizontal slide member 28. The two slide members of the upper/lower opposed pair of belt drives each include a rack portion 29 (one shown in Figure 4) which is operatively associated with an idler pinion 30.

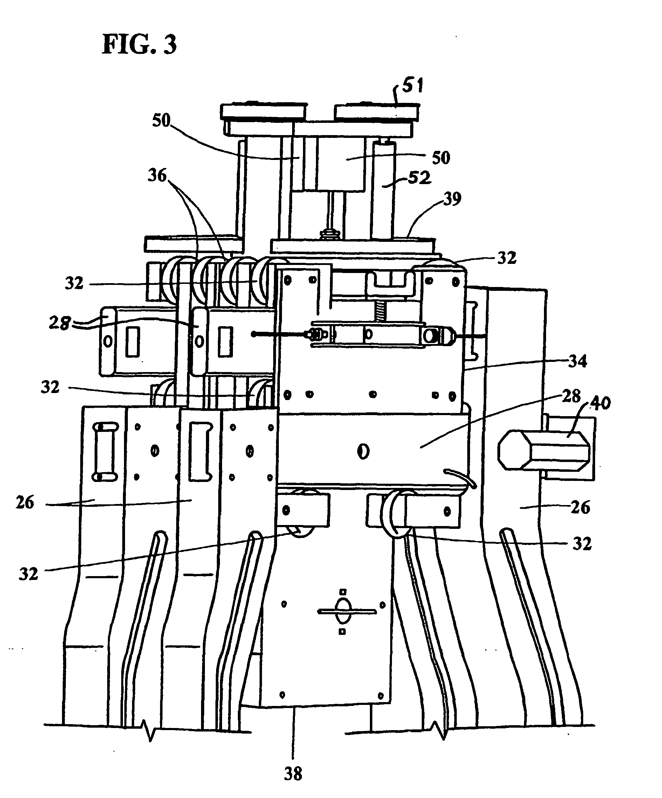

The two slide members 28 of an opposed pair of belt drives are supported for lateral displacement by three pairs of rollers 32 (Figures 3 and 4) which are mounted on a vertically displaceable carriage 34. The top slide member is supported between the top and middle roller pair and the bottom slide member (shown in Figure 3 but omitted from Figure 4 from below the lower rack portion 5 to reveal otherwise concealed components) is supported between the middle and bottom roller pairs. A slide member 28 can enter a suitable hole 31 in an associated opposed strut 26 when the opposed pair of belt drives is closed towards each other. The carriage 34 also supports upper and lower opposed pairs of rollers 36 which are captured by outer tracks (not shown) on a housing 38 (Figure 4) which is secured to the machine frame to depend over the inspection area, thus mounting the carriage for vertical displacement relative to the housing 38.

When an open/close step motor 40 is operated (there is a motor for each opposed pair of belt drives), a pulley 42 drives a screw 44 which is threadedly received by a corresponding nut secured within the lower slide member (not shown in Figure 4) which displaces the lower slide member and the attached strut. This displacement will be mirrored by the associated upper slide member and its opposed belt drive, since the upper and lower rack portions of the slide members are coupled together via the idler pinion 30. Operation of an open/close motor will accordingly conjointly horizontally displace the associated opposed upper or lower belt drives either towards or away from each other.

In addition to, or instead of, providing the motors 40 and screws 44 to displace the belt drives towards and/or away from each other, the displacement may be effected or supplemented by driving the associated pinions 30.

The housing 38 is integral with a mounting 39 which is secured to the machine frame, and mounted on the housing are a pair of up/down motors 50. One motor is associated with the upper drive belts and the other is associated with the lower drive belts. Each motor 50 operates a pulley 51 which rotatably drives a screw 53 which is operatively received by an elongated nut 52 which is joined to the carriage 34 via a bracket 54.

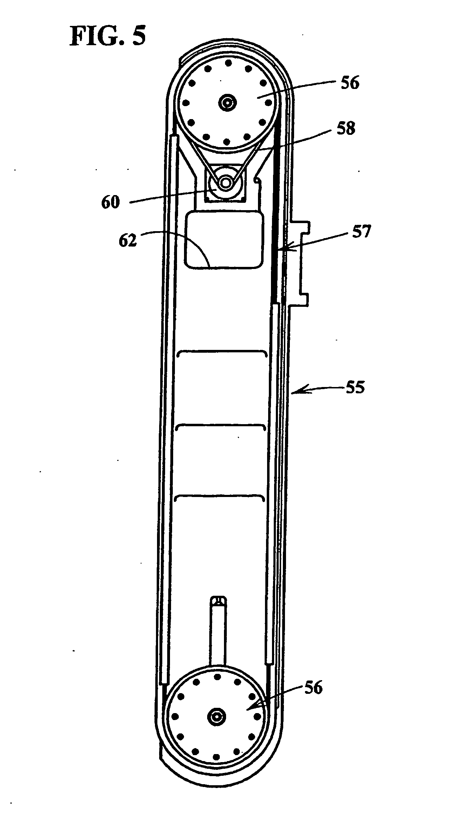

Each belt drive (Fig. 5) has a casing or arm 55 which supports a pulley 56 at either end of the arm. These pulleys support a belt 57 which is driven via a timing belt 58 interconnecting one of these pulleys with a step motor drive 60. The other pulley 56 is an idler pulley. The step motor is mounted on top of the casing as shown in Fig. 2. The step motor for a lower belt drive is mounted on the top surface of the lower casing so that it can enter a pocket or cutout 62 defined in the upper casing. To facilitate vertical displacement, each assembly is counterbalanced to substantially remove the weight of the assembly.

Figure 2 shows the upper and lower belt drive pairs being located in a large ware 12A configuration with the upper and lower belt drives being vertically stacked.

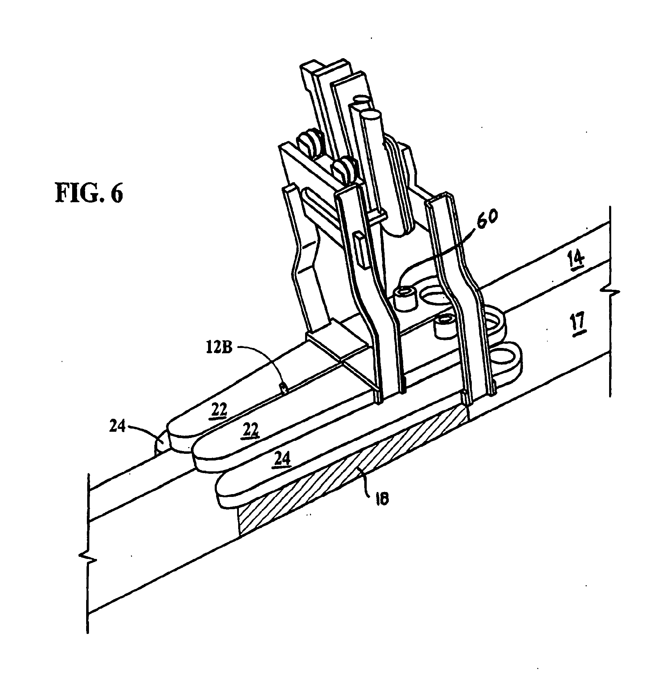

Figure 6 illustrates the location of the upper and lower drive belt pairs in a small or mini ware 12B configuration. To reach this configuration the upper belt drive pair is elevated to separate the vertically adjacent belt drives so that they can be displaced relatively horizontally (the top of the motor on the lower drive is clear of the upper casing). The lower belt drive pair is horizontally separated to the location where both lower belt drives are horizontally cleared of the front wall 17,18 and rear of the conveyor. The lower belt drives are then lowered until the top of the belt drive (its motor) is below the surface of the infeed/outfeed conveyors 14. Finally, the upper drive belt pair is now lowered to a location where the casing is just above the top surface of the conveyor 14. Now the drive belts of the upper drive belt pair can be located adjacent the sidewall of mini ware 12B.

## Claims

1. A belt drive conveyor system for a machine for inspecting containers, comprising
a belt drive conveyor mechanism (20) for picking up a container (12) at a pick up location (14) and delivering the picked up container to a release position (16),
said belt conveyor mechanism comprising upper (22) and lower (24) opposed pairs of belt drives, **characterised in that** said conveyor system further comprises
means (26) for supporting the upper and lower opposed pairs of belt drives so that
1. the upper drive belt pair (22) can be raised to a vertical location whereat there can be relative horizontal movement between the upper and lower belt drive pairs;
2. the lower belt drive pair (24) can be separated and lowered to an out of the way location; and
3. the upper belt drive pair (22) can then be lowered to be the only belt pair engaging a small container (12B) .

2. A belt drive conveyor system for a machine for inspecting containers according to claim 1, wherein each belt drive includes
a belt drive casing (55),
belt supporting means (56) mounted on said belt drive casing, said belt supporting means having a drivable member, and
motor means (60) mounted on said casing for driving said drivable member.

3. A belt drive conveyor system for a machine for inspecting containers according to claim 1 or 2, wherein said means for supporting the upper and lower opposed pairs of belt drives comprises
first support means for supporting the upper opposed pair (22) of belt drives cantilevered towards each other so that the belts of the opposed pair of belt drives can engage and drive a container (12),
second support means for supporting the lower opposed pair (24) of belt drives cantilevered towards each other so that the belts of the opposed pair of belt drives can engage and drive a container,
said first and second support means each including
a generally upwardly extending bracket (26) secured to each belt drive,
horizontal displacement means including a motor (40) for conjointly displacing said generally upwardly extending brackets (26) towards or away from each other to open or close said belt drives, and
vertical displacement means including a motor (50) for raising or lowering said horizontal displacement means.

## Patentansprüche

1. Riementriebförderersystem für eine Behälterprüfmaschine mit:
einer Riementrieb-Förderermechanik (20) zur Aufnahme eines Behälters (12) an einem Aufnahmeort (14) und zur Übergabe des aufgenommenen Behälters an einen Freigabeort (16);
wobei die Riemenförderermechanik obere (22) und untere (24) Paare gegenüberliegender Riementriebe aufweist,
**dadurch gekennzeichnet, dass** das Förderersystem weiterhin Einrichtungen (26) aufweist, durch die die oberen und unteren Paare gegenüberliegende Riementriebe derart lagerbar sind, dass
1. das obere Riementriebpaar (22) sich in eine vertikale Lage heben lässt, in der eine horizontale Relativbewegung zwischen den oberen und unteren Riementriebpaaren möglich ist;
2. das untere Riementriebpaar (24) sich trennen und in eine Lage außerhalb des Transportweges absenken lässt; und
3. das obere Riementriebpaar (22) sich dann absenken lässt, um das einzige Riemenpaar zu sein, das an einem kleinen Behälter (12B) anliegt.

2. Riementriebförderersystem für eine Behälterprüfmaschine nach Anspruch 1, bei dem jeder Riementrieb
ein Riementriebgehäuse (55),
eine Riemenlagerung (56), die auf dem Riementriebgehäuse angeordnet ist und ein antreibbares Element aufweist, und
eine Motoreinrichtung (60) aufweist, die auf dem Gehäuse angeordnet ist, um das antreibbare Element anzutreiben.

3. Riementriebförderersystem für eine Behälterprüfmaschine nach Anspruch 1 oder 2, bei dem die Einrichtungen zur Lagerung der oberen und unteren gegenüberliegenden Riementriebpaare
eine erste Lagereinrichtung zum Lagern des oberen Paares (22) gegenüberliegender Riementriebe derart, dass sie zueinander auskragen und die Riemen der gegenüberliegenden Riementriebpaare sich so an einen Behälter (12) anlegen und ihn antreiben können; und
eine zweite Lagereinrichtung zum Lagern des unteren Paares (24) gegenüberliegender Riementriebe aufweisen derart, dass sie zueinander auskragen und die Riemen der gegenüberliegenden Riementriebpaare sich so an einen Behälter anlegen und ihn antreiben können;
wobei die erste und die zweite Lagereinrichtung jeweils
einen allgemein aufwärts sich erstreckenden und an jedem Riementrieb befestigten Winkel (26),
eine horizontale Verschiebeeinrichtung mit einem Motor (40) zum gleichzeitigen Verschieben der allgemein aufwärts sich erstreckenden Winkel (26) zueinander hin oder voneinander weg, um die Riementriebe zu öffnen oder zu schließen, und
eine vertikale Verschiebbeeinrichtung mit einem Motor (50) aufweisen, um die horizontale Verschiebbeeinrichtung anzuheben oder abzusenken.

## Revendications

1. Système d'entraînement par courroie pour un dispositif d'inspection de récipients, comprenant
un mécanisme d'entraînement par courroie (20) pour ramasser un récipient (12) à un emplacement de ramassage (14) et délivrer le récipient ramassé à une position relâchée (16),
ledit mécanisme d'entraînement par courroie comprenant des paires opposées supérieure (22) et inférieure (24) de transmissions par courroie, et
**caractérisé en ce que** ledit système d'entraînement comprend en outre
des moyens (26) destinés à supporter les paires opposées supérieure et inférieure de transmissions par courroie de sorte que
1. la paire supérieure de transmission par courroie (22) puisse être relevée à un emplacement vertical après quoi il peut y avoir un mouvement horizontal relatif entre les paires supérieure et inférieure de transmissions par courroie ;
2. la paire inférieure de transmission par courroie (24) puisse être séparée et abaissée vers un emplacement isolé ; et
3. la paire supérieure de transmission par courroie (22) puisse ensuite être abaissée pour être la seule paire à courroie entrant en prise avec un petit récipient (12B).

2. Système d'entraînement par courroie pour un dispositif d'inspection de récipients selon la revendication 1, dans lequel chaque transmission par courroie comprend
un boîtier de transmission par courroie (55),
des moyens de support de courroie (56) montés sur ledit boîtier de transmission par courroie, lesdits moyens de support de courroie ayant un élément pouvant être entraîné, et
des moyens moteurs (60) montés sur ledit boîtier pour entraîner ledit élément entraînable.

3. Système d'entraînement par courroie pour un dispositif d'inspection de récipients selon la revendication 1 ou 2, dans lequel lesdits moyens de support des paires opposées supérieure et inférieure de transmissions par courroie comprennent
un premier moyen de support pour supporter la paire opposée supérieure (22) des transmissions par courroie en porte-à-faux l'une vers l'autre pour que les courroies de la paire opposée de transmissions par courroie puissent entrer en prise avec et entraîner un récipient (12),
un second moyen de support pour supporter la paire opposée inférieure (24) des transmissions par courroie en porte-à-faux l'une vers l'autre pour que les courroies de la paire opposée de transmissions par courroie puisse entrer en prise avec et entraîner un récipient,
lesdits premier et second moyens de support comprenant chacun
un support s'étendant généralement vers le haut (26) fixé sur chaque transmission par courroie,
des moyens de déplacement horizontal comprenant un moteur (40) pour déplacer conjointement lesdits supports s'étendant généralement vers le haut (26) vers ou à distance l'un de l'autre pour ouvrir ou fermer lesdites transmissions par courroie, et
des moyens de déplacement vertical comprenant un moteur (50) pour soulever ou abaisser lesdits moyens de déplacement horizontal.
